# FASCICULE DE BREVET EUROPEEN

(11) **EP 0 754 068 B1**
(45) Date de publication et mention de la délivrance du brevet: **05.12.2001**
(21) Numéro de dépôt: 95916722.2
(22) Date de dépôt: 06.04.1995
(51) Int. Cl.: A61M 5/30

(54) **DISPOSITIF D'INJECTION PAR JET SANS AIGUILLE**
NADELLOSE STRAHLINJEKTIONSVORRICHTUNG
NEEDLELESS JET INJECTION DEVICE

(30) Priorité: 06.04.1994 FR 9404041; 24.10.1994 FR 9412692
(43) Date de publication de la demande: 22.01.1997
(73) Titulaire: Moreau Defarges, Alain, 75016 Paris (FR); Moreau Defarges, Xavier, 78000 Versailles (FR)
(72) Inventeur: Moreau Defarges, Alain, 75016 Paris (FR); Moreau Defarges, Xavier, 78000 Versailles (FR)
(74) Mandataire: Armengaud Ainé, Alain
(86) Numéro de dépôt international: FR9500445
(87) Numéro de publication internationale: WO9527523

(56) Documents cités:
- EP-A- 0 338 806
- WO-A-88/09677
- US-A- 2 762 369
- US-A- 3 202 151
- US-A- 4 966 581
- US-A- 5 062 830

## Description

La présente invention est relative à un dispositif permettant l'injection de produit, notamment pharmaceutique, dans le corps humain ou animal.

Elle vise plus particulièrement un appareil dépourvu d'aiguille hypodermique, équipé d'un système armement, qui autorise l'administration par voie cutanée ou musculaire de substances médicamenteuses ou vaccinales, contenues dans une dose, à usage unique, formant cartouche.

On connaît des appareils d'injection par jet transcutané sans aiguille, qui ont été développés pour la médecine vétérinaire ou humaine. L'absence d'aiguille simplifie l'utilisation de l'appareil et ne requiert pas de connaissances spécifiques de par l'usager. Ils sont généralement utilisés pour des campagnes massives de vaccination et sont conçus de manière à mettre mis en oeuvre rapidement par un personnel non spécialiste. Le recours à un jet évite au maximum la contamination bactérienne et/ou virale d'un sujet à l'autre, qui se produisait dans les cas où une même aiguille est utilisée sans stérilisation pour plusieurs sujets. Ces appareils comme ceux visés dans le brevet US-A-3 202 151 sont généralement conformés sous forme de pistolet, équipé d'un récipient de produit à injecter, véhiculé au travers d'une buse, par l'action d'un piston se déplaçant dans une chambre, préalablement remplie de la substance, le piston étant mû par un percuteur ou comporte une culasse ou magasin renfermant une cartouche placée également dans l'axe du percuteur.

Le document US-A-2 762 369 donne un autre exemple de dispositif d'injection à culasse. La cartouche est une ampoule jetable remplie au préalable et qui doit être insérée dans la culasse.

Cette dernière est ensuite fixée à l'extrémité d'un appareil d'injection. On connaît également le document US-A-4 966 581, qui décrit lui aussi un système dans lequel une ampoule à usage unique doit être insérée dans une culasse, qui selon ce document, constitue l'extrémité de l'appareil d'injection.

Compte tenu du mode d'action du percuteur qui possède une énergie cinétique importante avant d'atteindre le piston de la cartouche qui peut, dans le cas où la cartouche n'est pas correctement disposée dans le magasin du pistolet, provoquer son éclatement au début de l'injection, ces appareils ne sont pas fiables et ne sont pas d'une mise en oeuvre aisée pour une utilisation ne relevant pas d'un usage fréquent.

On connaît par ailleurs le document US-A-5 062 830 qui vise un dispositif pourvu d'une cartouche à usage unique se fixant au corps d'un appareil d'injection sans culasse. Cette cartouche est vide lorsqu'elle est fixée à l'appareil d'injection. L'appareil d'injection doit donc également permettre le remplissage de la cartouche et comporte pour ce faire des moyens de fixation de son organe de percussion au piston de la cartouche, pour faire remonter celui-ci et créer une dépression dans la cartouche permettant son remplissage. Les moyens de fixation de l'organe de percussion au piston de la cartouche sont tels que, lorsque le piston de la cartouche atteint sa position haute, l'organe de percussion s'en détache et continue sa course vers le haut jusqu'à sa position d'armement. En effet, dans ce système comme dans ceux précédemment décrits, l'organe de percussion doit avoir une énergie suffisante avant de venir frapper le piston de la cartouche. La manipulation de ce système nécessite donc, outre la fixation de la cartouche, son remplissage, puis l'armement de l'organe de percussion avant l'injection proprement dite. De plus, l'asepsie n'est pas garantie du fait de la phase de remplissage.

La présente invention vise donc à remédier à ces inconvénients, en proposant un dispositif, dépourvu de magasin pour cartouche à usage unique et permettant l'injection par jet sans aiguille de produit préalablement contenu dans une cartouche, notamment en verre de type M1, directement placée en tête dudit dispositif, dans des conditions d'asepsie rigoureuses, pour un usage unique et individuel.

A cet effet, l'invention propose un dispositif par jet sans aiguille selon la revendication 1.

D'autres caractéristiques et avantages de la présente invention ressortiront de la description faite ci-après, en référence aux dessins annexés qui en illustrent un exemple de réalisation dépourvu de tout caractère limitatif. Sur les figures :

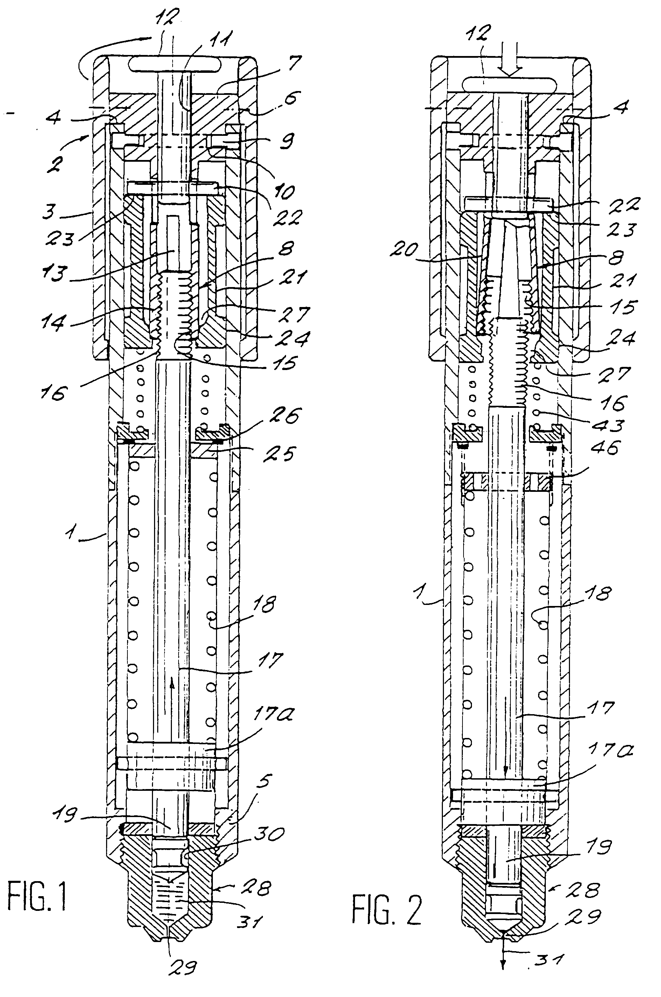

- la figure 1 est une vue en plan, en élévation frontale et en coupe, du dispositif selon l'invention, en position armée ;
- la figure 2 est une vue en plan, en élévation frontale et en coupe, du dispositif selon l'invention, en position déclenchée ;

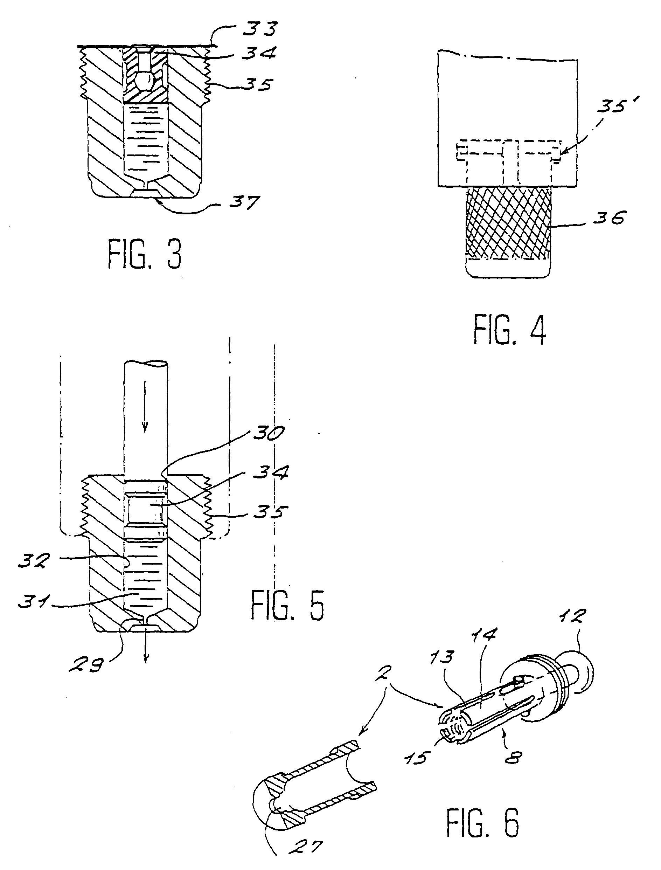

- la figure 3 est une vue à plus grande échelle et en coupe, d'une cartouche contenant une dose de produit injectable ;
- la figure 4 est une vue illustrant un autre mode de fixation de la cartouche sur le dispositif ;
- la figure 5 est une vue en coupe illustrant le mouvement du percuteur sur le bouchon en élastomère contenu dans la cartouche ;
- la figure 6 est une en perspective du système de pince devant emprisonner la tige du percuteur.

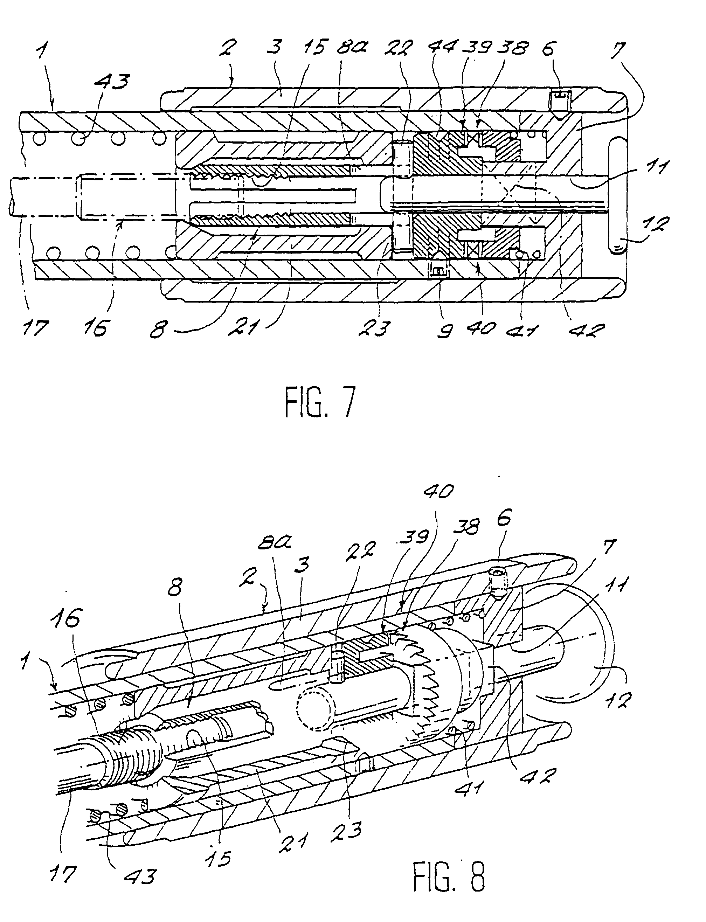

- la figure 7 est une vue en plan, en élévation frontale et en coupe, du dispositif selon un autre mode de l'invention, comportant un organe de liaison débrayable ;
- la figure 8 est une vue en perspective de l'organe de liaison débrayable.

Selon un mode préféré de réalisation, le dispositif objet de l'invention comprend essentiellement un corps 1 s'étendant sous la forme d'un tube, notamment de section droite cylindrique, destiné à recevoir un dispositif d'armement 2 lié à un capuchon 3, notamment moleté afin d'améliorer sa préhension par l'utilisateur, prévu à l'une des extrémités 4, 5 dudit corps. Le capuchon est réalisé à partir d'une portion de tube, de section similaire à celle du corps principal dudit dispositif, dont le diamètre est cependant supérieur afin d'en permettre un mouvement relatif de rotation autour du corps et coopère par l'intermédiaire d'un dispositif d'accrochage 6, notamment du type vis pointeau à une bague 7, elle-même solidaire d'une pince 8. Ladite bague est emmanchée avec jeu à l'extrémité du corps 1 et peut tourner librement dans ce dernier, sa position axiale dans le tube étant déterminée grâce à une butée radiale 9 à la paroi du tube et débouchant dans une rainure circulaire 10 pratiquée sur ladite bague 7 et positionnée en regard de la butée 9. Le mouvement de rotation issu du capuchon 3 est transmis par l'intermédiaire du dispositif d'accrochage 6 à la bague.

Ladite bague 7 est pourvue en outre d'un évidement central 11 pour le passage d'un percuteur 12, libre en translation axiale et débouchant à l'intérieur de ladite pince 8. Cette pince constitue l'un des maillons du dispositif d'armement. De forme globalement cylindrique, elle est munie d'une pluralité de découpes 13 orientées sensiblement parallèlement à l'axe du corps de manière à conformer une pluralité de pattes flexibles 14. On prévoit en outre de tarauder l'alésage 15 intérieur de ladite pince 8, de manière à conformer des empreintes aptes à se visser autour d'une portion filetée 16 prévue sur une tige 17 formant percuteur. Le pas du filetage est déterminé en fonction de la démultiplication choisie et de la constante de raideur d'un ressort 18. L'intensité du percuteur peut également être ajustée à l'aide d'un pré-réglage de l'écrasement du ressort, l'espace inter-spire pouvant être réglé par l'intermédiaire d'une molette 46 se déplaçant sur la tige 17 et en contact de 18 ou par l'intermédiaire d'un empilement de rondelles disposées entre le ressort 18 et d'une bague 25. Les spires du ressort 18 sont comprimées par le mouvement relatif de rotation entre le capuchon 3 et le corps 1.

On dispose donc une tige 17, formant piston, à l'intérieur dudit corps 1, sa longueur étant choisie de manière telle que son extrémité 19, non liée à ladite pince 8, affleure l'une des surfaces frontales 4, 5 du corps 1, lorsque le dispositif d'armement 2 est opérationnel.

L'enveloppe 20 périphérique de la pince est enserrée par une entretoise 21, également de section similaire à celle du corps, dont le diamètre extérieur correspond au diamètre intérieur du corps, de manière à conformer des zones de guidage. Sa position axiale à l'intérieur du corps étant limitée d'une part, à l'une de ses extrémités 23, 24, par une goupille 22 traversant de part en part et radialement le percuteur 12, et d'autre part son autre extrémité frontale constituant une surface d'appui pour un ressort 43 ; la position axiale du ressort et son autre surface d'appui étant en outre limitées par exemple par une bague 25 insérée dans le corps, solidaire de ladite tige 17 et immobilisée axialement éventuellement par un circlips 26, ou par un épaulement pratiqué sur la tige.

En position armement, l'utilisateur imprime un mouvement relatif de rotation entre le corps 1 et le capuchon 3 ; comme nous l'avons vu précédemment, le mouvement de rotation du capuchon se transmet à la fois à ladite bague 7 et à la pince 8 solidaire de cette dernière ; les quelques filets de ladite pince 8 en prise avec ceux de la tige permettent d'initier le mouvement de translation entre la tige 17 (formant vis) et la pince 8 (formant écrou) et le rapprochement relatif de ces deux pièces. Ce mouvement de translation comprime les spires du ressort 18 jusqu'à ce que l'espace inter-spires soit réduit au maximum. Selon un autre mode de réalisation, le ressort à spires peut être remplacé par un ressort hélicoïdal ou par tout autre dispositif élastique.

En position désarmement ou déclenchement, l'utilisateur imprime une légère poussée sur le percuteur 12, la goupille 22 faisant saillie radialement et au contact de l'une des faces frontales 23, 24 de l'entretoise, transmet un mouvement relatif de translation entre l'entretoise 21 et la pince 8, ce qui dégage l'extrémité de celle-ci d'une portée conique 27 prévue au fond de l'alésage de ladite entretoise 21. La poussée du ressort 18 combinée à la flexibilité des pattes de la pince libère les filets respectifs préalablement en prise, provoquant ainsi un mouvement de translation, quasiment impulsionnel de l'ensemble de la tige.

Selon un autre mode de réalisation de l'invention, on interpose, entre le capuchon 3 et l'organe d'armement solidaire de la pince 8 contenue dans le corps 1, un organe de liaison 38 ne permettant le mouvement relatif de rotation entre ces deux pièces que dans un seul sens de rotation. En effet, pour éviter toute détérioration de l'appareil et pour en simplifier la manipulation par l'utilisateur, on dispose d'un organe de liaison 38 débrayable se composant principalement de deux pièces 39, 40 notamment de section circulaire. L'une des pièces 39 est solidaire du capuchon 3, tandis que l'autre 40 est liée à la pince 8 et au corps 1 par l'intermédiaire d'une gorge 44, chacune des pièces dispose par ailleurs au niveau de leur face en contact d'une pluralité de zones 41 en relief. Ces zones 41 possèdent un profil apte à permettre d'une part un glissement relatif entre les pièces 39, et 40 selon un sens de rotation entre le capuchon 3 et le corps 1 et d'autre part à transmettre le couple dans l'autre sens de rotation du capuchon 3 par rapport au corps 1.

Préférentiellement, les zones 41 en relief sont réalisées à partir d'une pluralité de dents notamment à section droite triangulaire.

Pour que l'organe de liaison 38 fonctionne, il convient cependant de ne pas supprimer totalement les mouvements des pièces 39 et 40 par rapport au capuchon 3 et à la pince 8 ; ainsi lorsque l'organe de liaison est actif (débrayé) il faut que les pièces 39, 40 s'échappent longitudinalement l'une par rapport à l'autre et que ces pièces reviennent dans leur position d'engrènement après suppression du couple; on interpose donc au droit de la surface extérieure de l'une des pièces 39 ou 40 un organe élastique 41, notamment du type ressort, au niveau d'une portion de guidage 42, qui compense les mouvements de translation entre les pièces.

Selon une autre caractéristique de l'invention, on dispose à l'autre extrémité 5 du corps 1 dudit dispositif, une cartouche 28 par des moyens connus tels que notamment par vissage, par enclipsage (douille, baïonnette).

La cartouche 28, réalisée en matière plastique, métallique ou en verre, et globalement de forme sensiblement cylindrique, comporte un orifice 29, 30 à chaque bout. L'un 30 des orifices possède un diamètre sensiblement équivalent au diamètre de la tige 17, tandis que l'autre 29, de petit diamètre, notamment de l'ordre de quelques dixièmes de millimètre, sert de buse. La cavité interne de ladite cartouche est remplie sous vide d'un principe actif 31 et est éventuellement revêtue par un film 32 de matière, compatible avec les propriétés physico-chimiques dudit produit, afin de limiter au maximum le phénomène d'adsorption, la cartouche 28 ainsi obtenue est d'un usage unique et est donc jetable.

On prévoit par ailleurs d'interposer entre la dose de produit contenue dans la cavité de la cartouche et l'environnement un piston en élastomère 34 pour la fermeture de la dose, celui-ci devant communiquer au liquide la pression exercée par la tige 17 formant percuteur dans son extrémité 19. Ce piston en élastomère est recouvert, au niveau de l'orifice 30 de la cartouche, par un opercule 33 afin de garantir des conditions satisfaisantes d'asepsie. Cet opercule est enlevé au moment de l'installation de la cartouche 28 sur le dispositif.

L'enveloppe extérieure de ladite cartouche dispose par ailleurs, d'une part de moyens de fixation 35, 35' audit corps (pas de vis, picot...), et d'autre part de zones en relief 36 pour sa préhension par l'utilisateur. La face frontale correspondant à la buse comprend éventuellement une cuvette 37 dont la profondeur est variable mais garantit que le jet sortant de l'orifice de la buse possède le temps nécessaire à son établissement hydrodynamique avant l'injection unique sous-cutanée, intradermique ou intramusculaire.

L'invention telle qu'elle est décrite ci-dessus est d'une grande facilité d'emploi : il n'y a plus de stérilisation, ni de lavage de l'appareil, tout en garantissant une sûreté accrue pour l'utilisateur de par l'absence d'aiguille et l'impossibilité d'exécuter une réutilisation sans avoir préalablement rechargé par une nouvelle dose dans l'appareil, le mode de fixation de la cartouche évitant tous les risques d'éclatement de cette dernière de par l'absence de culasse. Cette invention est avantageusement adaptée à un usage par un utilisateur individuel ne possédant pas de connaissances spécifiques en matière d'injections sous-cutanées, intradermiques ou intramusculaires, elle réduit d'une part les risques d'accidents, toujours possibles avec un moyen d'injection à aiguille et d'autre part, elle supprime la peur de la piqûre ainsi que tout risque de contamination. L'utilisation de ce dispositif permet de plus de respecter la chronobiologie du patient. Cette invention trouve des développements avantageux dans l'injection d'une dose de faible volume, pouvant atteindre notamment de 0,05 à 0,2 ml. Elle présente un intérêt tout particulier pour l'administration de médicaments ou de vaccins chez l'homme ou l'animal. On citera, entre autres produits, des polypeptides ou des peptides, tels que des enzymes et tout spécialement la calcitonine, utilisée pour la prévention de la perte osseuse et le traitement de l'ostéoporose ou encore des médicaments contre la migraine. D'autres produits, en particulier des polypeptides ou des peptides, administrables par le dispositif objet de l'invention, comprennent des hormones, comme l'insuline, la somatostatine, l'hormone de croissance, des facteurs de la coagulation, par exemple des facteurs anti-hémophiliques, des composants du plasma, comme l'erythropoïétine, des polypeptides antiviraux, comme les interférons ou des immuno-modulateurs, comme les lymphokines. Ce dispositif est également spécialement approprié pour l'administration de préparations vaccinales.

Il demeure bien entendu que la présente invention n'est pas limitée aux exemples de réalisation décrits et représentés ci-dessus.

## Revendications

1. Dispositif d'injection d'un produit actif (31) par jet sans aiguille, comportant d'une part un appareil comportant un corps (1) tubulaire recevant un organe de .percussion (17) apte à effectuer un déplacement translatif axial quasiment impulsionnel, et d'autre part une cartouche (28) à usage unique comprenant : une cavité interne contenant le produit actif (31) à injecter, un premier orifice (29) servant de buse à une première extrémité axiale de la cartouche (28), un second orifice (30) disposé axialement à l'opposé du premier orifice (29), la cartouche étant munie d'un piston (34) s'interposant entre le produit actif (31) et l'environnement en fermant la cavité du côté du second orifice (30), le piston (34) étant apte à communiquer au produit actif (31) une pression exercée sur sa face opposée à la cavité interne et à se déplacer axialement dans la cavité vers le premier orifice et la cartouche (28) contenant ledit produit actif (31) étant apte à prendre une première position où elle n'est pas fixée audit appareil et comprenant en outre des moyens de fixation (35,35') aptes à coopérer avec le corps (1) de l'appareil d'injection de façon à ce que la cartouche contenant le produit actif soit apte à passer de ladite première position à une seconde position où elle est fixée par ledits moyens de fixation (35, 35') à une extrémité (5) du corps (1) dudit appareil dans l'axe de déplacement de l'organe de percussion (17) et à ce que dans cette deuxième position l'organe de percussion (17) puisse venir frapper ledit piston (34) pour y exercer ladite pression.

2. Dispositif selon la revendication 1, dans lequel le corps (1) est tubulaire, définissant ainsi l'extrémité (5) à laquelle est fixée la cartouche (28) dans ladite deuxième position, ainsi qu'une deuxième extrémité (4) opposée à la première, une bague (7) placée à ladite deuxième extrémité (4) et solidarisée par un système d'accrochage (6) à un capuchon (3) tubulaire placé à ladite deuxième extrémité (4), la bague (7) et le capuchon étant libres en rotation par rapport au corps (1), le corps (1) recevant un dispositif d'armement (2) comprenant une pince (8) munie d'une pluralité de découpes (13) orientées sensiblement parallèlement à l'axe du corps, conformant ainsi une pluralité de pattes flexibles (14), ladite pince (8) présentant un alésage (15) intérieur taraudé constituant des empruntes aptes à coopérer autour d'une portion filetée (16) prévue sur une tige (17) formant l'organe de percussion (17), ladite pince (8) étant soit solidaire de la bague, soit liée à celle-ci par un organe de liaison (38) débrayable n'autorisant l'entraînement de la pince (8) par la bague (7) et le capuchon (3) que dans un seul sens de rotation de ces derniers par rapport au corps (1).

3. Dispositif selon la revendication 2, dans lequel l'organe de liaison (38) comporte deux pièces (39, 40) de section circulaire disposant au niveau de leur face en contact d'une pluralité de zones (41) en relief, ces zones (41) possédant un profil apte à permettre d'une part un glissement relatif entre les pièces (39), et (40) selon un sens de rotation entre le capuchon (3) et le corps (1) et d'autre part à transmettre le couple dans l'autre sens de rotation du capuchon (3) par rapport au corps (1).

4. Dispositif selon l'une quelconque des revendications 2 ou 3, dans lequel la bague (7) est pourvue d'un évidement central (11) pour le passage d'un percuteur (12), libre en translation axiale et débouchant à l'intérieur de ladite pince (8) et en ce que l'enveloppe (20) périphérique de la pince (8) est enserrée par une entretoise (21) dont le diamètre extérieur correspond au diamètre intérieur du corps, de manière à conformer des zones de guidage, la position axiale de l'entretoise (21) à l'intérieur du corps étant limitée d'une part, à l'une de ses extrémités (23, 24) par une goupille (22) traversant de part en part et radialement le percuteur (12) et apte à transmettre à l'entretoise (21) le mouvement de translation axial du percuteur (12), et d'autre part, son autre extrémité frontale constituant une surface d'appui pour un ressort (43) de rappel de l'entretoise (21) dans sa position de repos.

5. Dispositif selon la revendication 4, dans lequel l'intensité du percuteur est ajustée à l'aide d'un pré-réglage de l'écrasement d'un ressort de détente (18), par l'intermédiaire d'une molette (46) se déplaçant sur la tige (17) ou par l'intermédiaire d'un empilement de rondelles disposées entre le ressort de détente (18) et la bague (25).

6. Dispositif selon l'une quelconque des revendications 1 à 5, **caractérisé en ce que** la cartouche est munie d'un opercule (33) recouvrant le piston (34) et destiné à être enlevé avant l'installation de la cartouche (28) sur le corps (1).

7. Dispositif selon l'une quelconque des revendications 1 à 5, **caractérisé en ce que** la cartouche est réalisée en matière plastique, métallique ou en verre.

8. Dispositif selon l'une quelconque des revendications 1 à 5, **caractérisé en ce que** la cavité de la cartouche est revêtue d'un film (32) de matière apte à limiter au maximum le phénomène d'adsorption.

9. Dispositif selon l'une quelconque des revendications 1 à 5, **caractérisé en ce que** la cartouche est remplie sous vide dudit produit actif (31).

10. Dispositif selon l'une quelconque des revendications 1 à 5, **caractérisé en ce que** l'enveloppe (20) extérieure de la cartouche dispose de zones en relief (36) pour sa préhension par l'utilisateur.

11. Dispositif selon l'une quelconque des revendications 1 à 5, **caractérisé en ce que** la face frontale de la cartouche correspondant à la buse comprend une cuvette (37) dont la profondeur garantit que le jet sortant de l'orifice de la buse possède le temps nécessaire à son établissement hydrodynamique avant l'injection sous-cutanée, intradermique ou intramusculaire.

12. Dispositif selon l'une quelconque des revendications 1 à 5, **caractérisé en ce que** la cartouche contient une dose unique de médicament destinée à être administrée par injection sous-cutanée, intramusculaire ou intradermique.

13. Dispositif selon l'une quelconque des revendications 1 à 5, **caractérisé en ce que** la cartouche contient des polypeptides ou des peptides, tels que des enzymes, des hormones, des facteurs de la coagulation, des produits antiviraux.

14. Dispositif selon l'une quelconque des revendications 1 à 5, **caractérisé en ce que** la cartouche contient de la calcitonine.

15. Dispositif selon l'une quelconque des revendications 1 à 5, **caractérisé en ce que** la cartouche contient une préparation vaccinale.

## Patentansprüche

1. Nadellose Strahlinjektionsvorrichtung für ein aktives Produkt (31), die einerseits ein Gerät mit einem rohrförmigen Körper (1) zur Aufnahme eines Schlagorgans (17), das eine praktisch stoßartige axiale Translationsverschiebung bewirken kann, und andererseits eine Patrone (Ampulle) (28) für einmaligen Gebrauch aufweist, wobei letztere einen das zu injizierende Produkt (31) enthaltenden inneren Hohlraum, eine als Düse dienende erste Öffnung (29) an einem ersten axialen Ende der Patrone (28), und eine zweite Öffnung (30), die axial entgegengesetzt zur ersten Öffnung (29) angeordnet ist, aufweist und die Patrone mit einem zwischen dem aktiven Produkt (31) und der Umgebung angeordneten Kolben (34) versehen ist, der den Hohlraum auf der Seite der zweiten Öffnung (30) abschließt, wobei der Kolben (34) dem aktiven Produkt (31) einen auf seine dem Innenhohlraum entgegengesetzte Seite ausgeübten Druck weitergeben und sich axial im Hohlraum in Richtung auf die erste Öffnung verschieben kann und die das aktive Produkt (31) enthaltende Patrone (28) eine erste Position einnehmen kann, in der sie nicht am Gerät befestigt ist, und im übrigen Befestigungsmittel (35, 35') aufweist, die mit dem Körper (1) des Injektionsgerätes in der Weise zusammenwirken können, daß die das aktive Produkt enthaltende Patrone aus der genannten ersten Position in eine zweite Position gelangen kann, wo sie durch die Befestigungsmittel (35, 35') an einem Ende (5) des Körpers (1) des Geräts in der Verschiebungsachse des Schlagorgans (17) befestigt ist und in dieser zweiten Position das Schlagorgan (17) auf den Kolben (34) treffen kann, um dort den Druck auszuüben.

2. Vorrichtung nach Anspruch 1, worin der Körper (1) rohrförmig ist und so das Ende (5) definiert, an dem die Patrone (28) in der zweiten Stellung fixiert ist, sowie ein zweites, dem ersten entgegengesetztes Ende (4) definiert, ein Ring (7) an dem zweiten Ende (4) angeordnet und durch ein Verbindungssystem (6) mit einer am zweiten Ende (4) angeordneten rohrförmigen Kappe (3) fest verbunden ist, wobei der Ring (7) und die Kappe bezüglich des Körpers (1) frei drehbar sind, der Körper (1) eine Spannvorrichtung (2) aufnimmt, welche eine Klemmzange (8) aufweist, die mit einer Mehrzahl von im wesentlichen parallel zur Achse des Körpers ausgerichteten Ausschnitten (13) versehen ist, die so eine Mehrzahl von biegsamen Armen (14) bilden, die Klemmzange (8) eine innere Gewindebohrung (15) aufweist, welche Vertiefungen bildet, die ringsum mit einem Gewindeabschnitt (16) zusammenwirken, der an einer das Schlagorgan (17) bildenden Stange vorgesehen ist, wobei die Klemmzange (8) entweder mit dem Ring fest verbunden oder mit diesem mittels eines ausschaltbaren Verbindungselements (38) verbunden ist, das den Antrieb der Klemmzange (8) durch den Ring (7) und die Kappe (3) nur in einer Drehrichtung der beiden letzteren bezüglich des Körpers (1) zuläßt.

3. Vorrichtung nach Anspruch 2, worin das Verbindungselement (38) zwei Teile (39, 40) mit kreisförmigem Querschnitt aufweist, die auf der Höhe ihrer in Berührung kommenden Seite eine Mehrzahl von Reliefzonen (41) aufweisen, wobei diese Zonen (41) ein Profil besitzen, das einerseits in einer Drehrichtung zwischen der Kappe (3) und dem Körper (1) eine relative Gleitbewegung zwischen den Teilen (39) und (40) erlaubt und andererseits in der anderen Drehrichtung der Kappe (3) bezüglich des Körpers (1) das Drehmoment überträgt.

4. Vorrichtung nach einem der Ansprüche 2 oder 3, worin der Ring (7) mit einer zentralen Ausnehmung (11) für den Durchtritt eines Schlagbolzens (12) versehen ist, der axial frei verschiebbar ist und bis in das Innere der Klemmzange (8) reicht, und daß der Umfangsmantel (20) der Klemmzange (8) durch eine Zwischenwand (21) eingespannt ist, deren Außendurchmesser dem Innendurchmesser des Körpers entspricht, so daß Führungszonen gebildet werden, wobei die axiale Stellung der Zwischenwand (21) im Inneren des Körpers einerseits an einem ihrer Enden (23, 24) durch einen den Schlagbolzen (12) von der einen zur anderen Seite radial durchsetzenden Steckstift (22) begrenzt ist, der die axiale Verschiebungsbewegung des Schlagbolzens (12) auf die Zwischenwand (21) übertragen kann, und andererseits deren anderes Frontende eine Anschlagfläche für eine Feder (43) bildet, welche die Zwischenwand (21) in ihre Ruhestellung zurückstellt.

5. Vorrichtung nach Anspruch 4, worin die Wirkungsstärke des Schlagbolzens mit Hilfe einer Voreinstellung der Stauchung einer Entspannungsfeder (18) mittels einer auf der Stange (17) verschiebbaren Rädchens (46) oder mittels eines Stapels von zwischen der Entspannungsfeder (18) und dem Ring (25) angeordneten Unterlegscheiben einstellbar ist.

6. Vorrichtung nach einem der Ansprüche 1 bis 5, **dadurch gekennzeichnet, daß** die Patrone mit einem den Kolben (34) abdeckenden Hütchen (33) versehen ist, das vor dem Anbringen der Patrone (28) am Körper (1) abgenommen wird.

7. Vorrichtung nach einem der Ansprüche 1 bis 5, **dadurch gekennzeichnet, daß** die Patrone aus Kunststoff, Metall oder Glas hergestellt ist.

8. Vorrichtung nach einem der Ansprüche 1 bis 5, **dadurch gekennzeichnet, daß** die Patrone mit einem Film (32) aus einem Stoff überzogen ist, der eine Adsorption weitestgehend verhindert.

9. Vorrichtung nach einem der Ansprüche 1 bis 5, **dadurch gekennzeichnet, daß** die Patrone unter Vakuum mit dem aktiven Produkt (31) gefüllt ist.

10. Vorrichtung nach einem der Ansprüche 1 bis 5, **dadurch gekennzeichnet, daß** der Außenmantel (20) der Patrone Reliefzonen (36) aufweist, damit sie vom Benutzer leichter ergriffen werden kann.

11. Vorrichtung nach einem der Ansprüche 1 bis 5, **dadurch gekennzeichnet, daß** die der Düse entsprechende Frontseite der Patrone eine schalenförmige Vertiefung (37) aufweist, deren Tiefe garantiert, daß der aus der Öffnung der Düse austretende Strahl genügend Zeit hat, um vor der subkutanen, intradermalen oder intramuskulären Injektion seine hydrodynamischen Eigenschaften auszubilden.

12. Vorrichtung nach einem der Ansprüche 1 bis 5, **dadurch gekennzeichnet, daß** die Patrone eine Einzeldosis des durch subkutane, intramuskuläre oder intradermale Injektion zu verabreichenden Medikaments enthält.

13. Vorrichtung nach einem der Ansprüche 1 bis 5, **dadurch gekennzeichnet, daß** die Patrone Polypeptide oder Peptide, wie Enzyme, Hormone, Gerinnungsfaktoren, antivirale Produkte enthält.

14. Vorrichtung nach einem der Ansprüche 1 bis 5, **dadurch gekennzeichnet, daß** die Patrone Calcitonin enthält.

15. Vorrichtung nach einem der Ansprüche 1 bis 5, **dadurch gekennzeichnet, daß** die Patrone einen Impfstoff enthält.

## Claims

1. Needleless jet injection device for an active product (31) comprising on the one hand a device comprising a tubular body (1) receiving an impacting member (17) capable of performing an axial transverse almost pulse-like displacement, and on the other hand a disposable cartridge (28): an internal cavity containing the active product (31) to be injected, a first opening (29) acting as a nozzle at a first axial end of the cartridge (28), a second opening (30) disposed axially opposite the first opening (29), the cartridge being equipped with a piston (34) interposed between the active product (31) and the environment c!osing the cavity of the side of the second opening (30), the piston (34) being able to communicate to the active product (31) a pressure exerted on the opposite face of the internal cavity and to be displaced axially in the cavity towards the first opening and the cartridge (28) containing the said active product (31) being capable of adopting a first position in which it is not fixed to the said device, and also comprising securing means (35,35') able to cooperate with the body (1) of the injection device in such a way that the cartridge containing the active product is capable of passing from said first position to a second position, where it is secured by said securing means (35, 35') to one end (5) of the body (1) of said device in the axis of displacement of the impacting member (17) and in this second position the impacting member (17) can strike said piston (34) to exert the said pressure.

2. Device according to claim 1, wherein the body (1) is tubular, thus defining the end (5) to which the cartridge (28) is fixed in said second position, as well as a second end (4) opposite to the first, a ring (7) placed at said second end (4) and connected by a linking system (6) to a tubular cap (3) positioned at the said second end (4), the ring (7) and the cap being freely rotatable in relation to the body (1), the body (1) receiving a reinforcing device (2) comprising a clamp (8) provided with a plurality of slots (13) aligned almost parallel to the axis of the body, thus forming a plurality of flexible tabs (14), said clamp (8) having an internal bore (15) tapped so as to produce impressions adapted to cooperate around a threaded portion (16) provided on a rod (17) forming the impacting member (17), said clamp (8) being either connected to the ring or linked to the latter by a disengageable linking member (38) only permitting the entrainment of the clamp (8) by the ring (7) and the cap (3) in a single direction of rotation of the latter in relation to the body (1).

3. Device according to claim 2, wherein the linking member (38) comprises two members (39, 40) of circular section having at the level of their contacting face a plurality of zones (41) in relief, said zones (41) having a profile which is adapted on the one hand to allow a relative sliding between the members (39) and (40), in one direction of rotation between the cap (3) and the body (1) and on the other hand to transmit the torque in the other direction of rotation of the cap (3) in relation to the body (1).

4. Device according to any one of claims 2 or 3, wherein the ring (7) has a central cutaway (11) for the passage of a striking pin (12), making a free axial traversing movement and extending into the interior of said clamp (8), and the peripheral envelope (20) of the clamp (8) is clamped by a strut (21), whose external diameter corresponds to the internal diameter of the body, so as to produce guiding zones, the axial position of the strut (21) inside the body being limited at one of its ends (23, 24) by a pin (22) which extends radially right through the striking pin (12) and is capable of transmitting to the strut (21) the axial traversing movement of the striking pin (12), the other end face of said strut forming a bearing surface for a spring (43) which biases the strut (21) to its inoperative position.

5. Device according to claim 4, wherein the intensity of the striking pin is adjusted by the preadjustment of the compression of a trigger spring (18) by means of a roller (46) moving on the rod (17) or by means of stack of washers disposed between the trigger spring (18) and the ring (25).

6. Device according to any one of claims 1 to 5, **characterised in that** the cartridge is equipped with an inner capsule (33) covering the piston (34) and designed to be removed before the cartridge (28) is installed on the body (1).

7. Device according any one of claims 1 to 5, **characterised in that** the cartridge is made of plastic, metal or glass.

8. Device according to any one of claims 1 to 5, **characterised in that** the cavity of the cartridge is coated with a film (32) of material suitable for the maximum limitation of adsorption.

9. Device according to any one of claims 1 to 5, **characterised in that** the cartridge is filled under vacuum with said active product (31).

10. Device according to any one of claims 1 to 5, **characterised in that** the external envelope (20) of the cartridge has zones in relief (36) to be grasped by the user.

11. Device according to any one of claims 1 to 5, **characterised in that** the front face of the cartridge corresponding to the nozzle comprises a cuvette (37) the depth of which ensures that the jet emerging from the nozzle opening has enough time to become hydrodynamically established before subcutaneous, intradermal or intramusclar injection.

12. Device according to any one of claims 1 to 5, **characterised in that** the cartridge contains a single dose of medicine which is intended to be administered by subcutaneous, intramuscular or intradermal injection.

13. Device according to any one of claims 1 to 5, **characterised in that** the cartridge contains polypeptides or peptides such as enzymes, hormones, coagulation factors, antiviral products.

14. Device according to any one of claims 1 to 5, **characterised in that** the cartridge contains calcitonin.

15. Device according to any one of claims 1 to 5, **characterised in that** the cartridge contains a vaccine.
